**Europäisches Patentamt**

**European Patent Office**

**Office européen des brevets**

(19)

(11) Veröffentlichungsnummer : **0 455 029 B1**

(12) **EUROPÄISCHE PATENTSCHRIFT**

(45) Veröffentlichungstag der Patentschrift :
18.11.93 Patentblatt 93/46

(51) Int. Cl.$^5$ : **C10L 1/02**

(21) Anmeldenummer : **91105920.2**

(22) Anmeldetag : **13.04.91**

(54) **Verfahren zur Herstellung von hochoktanigen, olefinarmen Kraftstoffen und Kraftstoffkomponenten.**

(30) Priorität : **28.04.90 DE 4013711**

(43) Veröffentlichungstag der Anmeldung :
**06.11.91 Patentblatt 91/45**

(45) Bekanntmachung des Hinweises auf die
Patenterteilung :
**18.11.93 Patentblatt 93/46**

(84) Benannte Vertragsstaaten :
**AT BE DE FR GB NL**

(56) Entgegenhaltungen :
**EP-A- 0 197 348**
**DE-A- 3 526 443**

(73) Patentinhaber : **EC ERDÖLCHEMIE GMBH**
**Alte Strasse 201**
**D-50769 Köln (DE)**

(72) Erfinder : **Stüwe, Arnd, Dr.**
**Berta-von-Suttner-Strasse 34**
**W-5090 Leverkusen 1 (DE)**
Erfinder : **Tschorn, Herbert**
**Rembrandtstrasse 18**
**W-4047 Dormagen (DE)**
Erfinder : **Scheef, Hans-Volker**
**Goethestrasse 69**
**W-4047 Dormagen (DE)**
Erfinder : **Michel, Jörg-Uwe**
**Ostpreussenallee 10**
**W-4047 Dormagen (DE)**

(74) Vertreter : **Zobel, Manfred, Dr. et al**
**BAYER AG Konzernverwaltung RP Patente**
**Konzern**
**D-51368 Leverkusen (DE)**

## Beschreibung

Die vorliegende Erfindung betrifft ein Verfahren zur Herstellung von Kraftstoffen und Kraftstoffkomponenten aus Kohlenwasserstoffen, die tert.-Olefine im Gemisch mit gesättigten und olefinisch, diolefinisch und acetylenisch ungesättigten Kohlenwasserstoffen enthalten, wobei die tert.-Olefine mit einem oder mehreren $C_1$-$C_4$-Alkanolen verethert werden und die ungesättigten Bestandteile weitgehend hydriert werden.

Es ist lange bekannt, tert.-Olefine mit niederen Alkanolen zu Alkyl-tert.-alkyl-ethern umzusetzen. Es ist weiterhin bekannt, daß im Falle des Methyl-tert.-butyl-ethers (MTBE) das Gleichgewicht bei nicht zu hohen Temperaturen weitgehend auf der Seite des Ethers liegt, während bereits im Falle des tert.-Amyl-methyl-ethers (TAME) bei gleicher Temperatur wesentlich geringere Mengen an Ether vorliegen, dafür aber beträchtliche Mengen an den Ausgangsstoffen Methanol und tert.-Amylenen. Bei Ethern aus noch höheren tert.-Olefinen wird die Gleichgewichtseinstellung zu den Ethern noch ungünstiger. Gesichertes Fachwissen ist es ferner, daß solche Ether bei noch höheren Temperaturen wieder in die Ausgangsstoffe gespalten werden. Die Bildung und die Spaltung der Ether, also die Gleichgewichtseinstellung bei den jeweiligen Temperaturen werden von sauren Katalysatoren beeinflußt, die häufig genug die gleichen sowohl für die Bildung als auch für die Spaltung sein können, wobei jeweils die zur Bildung bzw. Zersetzung günstige Temperatur eingestellt wird.

Die selektive Veretherung der tert.-Olefine ist auch bereits genutzt worden, um Crackbenzine durch Bildung solcher Ether zu höheroktanigen Kraftstoffen und Kraftstoffkomponenten umzusetzen. Hierbei bleiben die einfach ungesättigten und die mehrfach ungesättigten Bestandteile des Crackbenzins in solchen Kraftstoffen bzw. Kraftstoffkomponenten.

Die hochungesättigten Bestandteile solcher Gemische sind Gum-Bildner. Unter Gum wird im Zusammenhang mit Kraftstoffen ein Gehalt an oligomeren oder polymeren Stoffen im Kraftstoff verstanden, der bei der Analytik der Kraftstoffe als Abdampfrückstand ausgewiesen wird. Ein Gehalt an Gum im Kraftstoff führt nicht nur zur Verkokung und zu Ablagerungen im Brennraum des Moters, sondern belegt schon während der Herstellung solcher Kraftstoffe die Poren eines Veretherungskatalysators, vermindert damit dessen Aktivität, verkürzt seine Lebensdauer und führt zusätzlich zur Gelbfärbung des entstehenden Kraftstoffs.

Es ist nun bereits ein Verfahren bekanntgeworden (DE-OS 35 38 564), das es gestattet, gleichzeitig zur Veretherung von in rohen Kohlenwasserstoffgemischen enthaltenen $C_5$-$C_8$-tert.-Olefinen mit $C_1$-$C_5$-Alkanolen durch eine Zugabe von geringen Mengen an Wasserstoff die in diesen rohen Kohlenwasserstoffgemischen enthaltenen hochungesättigten und stark reaktiven Anteile, die für die Gum-Bildung verantwortlich gemacht werden, zu hydrieren. Eine solche gleichzeitige Veretherung und Hydrierung unter milden Bedingungen wird an Styrol-divinylbenzolharsen durchgeführt, die freie Sulfonsäuregruppen und in metallischem Zustand befindliche Elemente der VI., VII. oder VIII. Nebengruppe des Periodensystems der Elemente enthalten.

Bei einer solchen gleichzeitigen Veretherung und Teilhydrierung der hochungesättigten Gum-Bildner verbleiben die Monoolefine im Reaktionsgemisch. Diese Monoolefine sind nur unter verschärften Reaktionsbedingungen zu den zugehörigen Paraffinen zu hydrieren. Eine solche Hydrierung ist sehr wünschenswert, da die Monoolefine als Radikalbildner neben anderen Stoffen für die Schädigung der Ozonschicht der oberen Atmosphäre verantwortlich gemacht werden. Ein weiterhin anhaltender großer Anteil von Olefinen in den weltweit in großem Maßstab gebrauchten Motorkraftstoffen muß daher nach heutigem Verständnis in verstärktem Maße zur unerwünschten weiteren Schädigung dieser Ozonschicht beitragen. Es hat daher nicht an Anstrengungen gefehlt, die Olefine in den Kraftstoffen soweit wie möglich durch Hydrierung in die zugehörigen Paraffine umzuwandeln. Da nach fachmännischem Wissen eine Hydrierung der Monoolefine unter verschärften Bedingungen, wie erhöhte Temperatur, erhöhtem Wasserstoffangebot und erhöhtem Druck durchzuführen war, mußte stets mit einer Zerstörung der Oktanzahl erhöhenden und daher gewünschten Ether aus den tert.-Olefinen gerechnet werden. Eine solche Zerstörung war um so mehr zu befürchten, als auch die Mitwirkung der häufig sauer eingestellten Katalysatorträger der einzusetzenden Hydrierkatalysatoren in Betracht gezogen werden mußte. Wirksame Hydrierverfahren für die Monoolefine unter Erhaltung der Alkyl-tert.-alkyl-ether mußten daher unter besonderen Bedingungen ablaufen. So ist in DE-OS 35 26 443 ein Hydrierverfahren für olefinische Kohlenwasserstoffe, die sich im Gemisch mit Alkyl-tert.-alkyl-ethern befinden, beschrieben, wobei sehr spezielle Katalysatoren verwendet werden, die eine hydrieraktive Komponente auf einem Katalysatorträger mit einer spezifischen Oberfläche von mehr als 50 m²/g und einem Porendurchmesser von überwiegend <1000 nm besitzen.

Aus dem fachmännischen Verständnis heraus und in Kenntnis der bisherigen Anstrengungen zur erfolgreichen Durchführung der Veretherung einerseits und der weitgehenden Hydrierung der Olefine unter Erhalt der bereits gebildeten Ether andererseits war nicht zu erwarten, daß über die leicht durchzuführende partielle Hydrierung nur der hochungesättigten Verbindungen hinaus während der Veretherung auch die weitgehende Hydrierung der schwerer zu hydrierenden Monoolefine durchgeführt werden könnte, wobei folgende Forderungen erfüllt sein mußten:

1. die vorhandenen Monoolefine ohne tertiäres C-Atom sollten weitgehend hydriert werden, ohne daß die zu verethernden tert.-Olefine hydriert werden, so daß die maximal mögliche Ausbeute an Alkyl-tert.-alkylether erreicht wird;

2. die verschärften Reaktionsbedingungen, zu denen auch eine gegenüber der partiellen Hydrierung erhöhte Hydriertemperatur gehört, sollten nicht zu einer Spaltung einmal gebildeter Ether in die Ausgangsprodukte Alkanol und tert.-Olefin führen;

3. die stark exotherme Reaktionswärme der Hydrierung sollte nicht zu Überhitzungen des Reaktionsgemisches führen, die die unter 2. angeführten zu befürchtenden Tendenzen verstärken würde.

Es wurde nun überraschend gefunden, daß entgegen den bisherigen Befürchtungen eine Erhöhung des Wasserstoffangebots unter erhöhtem Druck die Veretherung der tert.-Olefine nicht beeinträchtigt und daß eine unerwünschte Hydrierung der zu verethernden tert.-Olefine bei einer gleichzeitig durchgeführten Veretherung und Hydrierung praktisch vollständig unterbleibt.

Die Erfindung betrifft daher ein Verfahren zur Herstellung von hochoktanigen, olefinarmen Kraftstoffen und Kraftstoffkomponenten mit einem Gehalt an Alkyl-tert.-alkyl-ethern, das dadurch gekennzeichnet ist, daß man einen rohen Kohlenwasserstoffstrom, der $C_5$-$C_8$-tert.- Olefine, bevorzugt $C_5$-$C_7$-tert.-Olefine, neben gesättigten und olefinisch, diolefinisch und acetylenisch ungesättigten Kohlenwasserstoffen im gleichen Siedebereich enthält, gleichzeitig mit einem oder mehreren $C_1$-$C_4$-Alkanolen, bevorzugt $C_1$-$C_2$-Alkanolen, besonders bevorzugt Methanol, in einer Menge von 0,5 bis 5 Mol, bevorzugt 0,8 bis 2,5 Mol, besonders bevorzugt 1 bis 2 Mol, pro Mol des oder der tert.-Olefine und mit 1,5 bis 20 Mol pro Mol der Gesamtungesattigheit an Wasserstoff unter einem Druck von 12 bis 80 bar, bevorzugt 15 bis 60 bar, besonders bevorzugt 15 bis 35 bar, und bei einer Temperatur von 60 bis 180°C, bevorzugt 70 bis 140°C, besonders bevorzugt 70 bis 120°C, an einem an sich bekannten Katalysator oder einem Gemisch an sich bekannter Katalysatoren, der oder das sowohl die Veretherung als auch die Olefinhydrierung bewirkt, umsetzt.

Das erfindungsgemäße Verfahren ist durch die folgenden vorteilhaften Wirkungen ausgezeichnet:

1. die hochungesättigten Gum-Bildner werden durch Hydrierung eliminiert und finden sich somit weder im Kraftstoff, noch können sie den oder die eingesetzten Katalysator(en) durch Belegung desaktivieren;

2. die durch Veretherung der tert.-Olefine entstehenden Ether erhöhen die Klopffestigkeit der entstehenden Kraftstoffe (Oktanzahlerhöhung);

3. der Gehalt an Monoolefinen wird sehr stark abgesenkt, wobei schädliche Auswirkungen auf die Umwelt ausgemerzt werden;

4. die Verringerung sowohl der hochungesättigten als auch der monoolefinisch ungesättigten Verbindungen ergibt niedrigere Farbzahlen im erhaltenen Kraftstoff;

5. die aus den ungesättigten Verbindungen entstehenden Paraffine zeichnen sich durch einen niedrigeren Dampfdruck, verglichen mit den ursprünglich ungesättigten Verbindungen, aus, was wiederum aus Umweltschutzgründen zu einer wünschenswerten geringeren Flüchtigkeit führt;

6. die weitgehende Verringerung aller ungesättigten Verbindungen ergibt weiterhin eine wünschenswerte Verringerung des Sensitivity, d.h. eine Verringerung des Abstandes zwischen der Motor-Oktanzahl (MOZ) und der Research-Oktanzahl (ROZ). Die Sensitivity sinkt von Werten von etwa 18 bis 20 auf Werte von etwa 10 bis 12.

Als tert.-Olefine seien beispielsweise solche genannt, die 5 bis 8, bevorzugt 5 bis 7 C-Atome enthalten; besonders bevorzugt sind $C_5$- und/oder $C_6$-tert.-Olefine. Solche tert.-Olefine sind in den erfindungsgemäß umzusetzenden rohen Kohlenwasserstoffströmen im wesentlichen als Gemisch mehrerer von ihnen enthalten. Weiterhin können solche Kohlenwasserstoffströme stets gesättigte oder ungesättigte Kohlenwasserstoffe mit benachbarten C-Zahlen enthalten. Beispielsweise enthält ein Kohlenwasserstoffgemisch, das im wesentlichen tert.-Amylene enthält, im allgemeinen geringe Mengen an i-Buten und/oder tert.-Hexenen. Als nicht tertiäre Olefine sind beispielsweise $C_5$-$C_9$-Olefine geradkettiger, cyclischer und/oder verzweigter Art enthalten, bevorzugt jene, die 5 bis 7 C-Atome enthalten, besonders bevorzugt wiederum solche die $C_5$- und/oder $C_6$-Olefine darstellen. Auch diese Olefine sind in den erfindungsgemäß umzusetzenden rohen Kohlenwasserstoffgemischen im wesentlichen als Gemisch mehrerer von ihnen enthalten. Beispielsweise enthält ein Kohlenwasserstoffstrom mit tert.-Amylenen ebenso auch n-Pentene und Cyclopenten. Weiterhin enthalten solche rohen Kohlenwasserstoffgemische Bestandteile, die unter den Umsetzungsbedingungen oligomere oder polymere Stoffe bilden, die dem Fachmann als Gum bekannt sind. Bei diesen Gum-Bildnern handelt es sich beispielsweise um Diolefine und/oder acetylenische und/oder olefinisch-acetylenische Verbindungen. Gleichfalls vergesellschaftet mit den genannten ungesättigten Verbindungen finden sich auch die gesättigten geradkettigen oder verzweigten oder cyclischen Kohlenwasserstoffe des gleichen Siedebereichs.

Kohlenwasserstoffe der genannten Art stehen in petrochemischen Anlagen und/oder Raffinerien zur Verfügung. Sie können beispielsweise bei der Umsetzung von Naphtha, Liquid Petroleum Gas (LPG), Rohöldestillaten, Gasölen oder anderen Ausgangskohlenwasserstoffgemischen in Steam-Crackern, katalytischen Crackern, Isomerisierungs- oder Dehydrierungsanlagen gewonnen werden. Sie können als solche mit einer größeren Breite an C-Atomzahlen oder als engere Destillationsschnitte erfindungsgemäß eingesetzt werden, wobei beispielsweise die im wesentlichen $C_5$- oder $C_6$-Kohlenwasserstoffe enthaltenen Destillationsschnitte geringe Anteile der sich anschließenden $C_4$- und/oder $C_7$-Homologen enthalten, Typische Zusammensetzungen für $C_5$-Destillationsschnitte aus Steam-Crackern oder katalytischen Crackern sind etwa die folgenden:

| Stoff, Gew.-% (ca.) | Steamcracker | | | Cat. Cracker | | |
|---|---|---|---|---|---|---|
| $C_4$/Leichte | 1 | - | 5 | 1 | - | 5 |
| n-/i-Pentan | 20 | - | 30 | 30 | - | 35 |
| n-Pentene | 15 | - | 20 | 25 | - | 30 |
| 3-Methyl-buten-1 | 0,5 | - | 3 | 0,5 | - | 2 |
| 2-Methyl-buten-1 | 5 | - | 10 | 7 | - | 11 |
| 2-Methyl-buten-2 | 8 | - | 17 | 15 | - | 20 |
| Cyclopentan | 4 | - | 6 | 1 | - | 3 |
| Cyclopenten | 20 | - | 27 | 2 | - | 5 |
| Diolefine/Acetylene | 0,5 | - | 5 | 0,5 | - | 5 |

Ein typischer Kohlenwasserstoffstrom für das erfindungsgemäße Verfahren ist der nach der Abtrennung der $C_4$-Kohlenwasserstoffe und vor der Gewinnung von Aromaten entstehende sogenannte Benzol-Vorlauf.

Als Alkanole für das erfindungsgemäße Verfahren seien beispielsweise primäre oder sekundäre, bevorzugt primäre Alkanole mit 1 bis 4, bevorzugt 1 bis 2 C-Atomen genannt. Ganz besonders bevorzugt ist der Einsatz von Methanol. Die Alkanole werden in einer Menge von 0,5 bis 5 Mol, bevorzugt 0,8 bis 2,5 Mol, besonders bevorzugt 1 bis 2 Mol pro Mol des oder der tert.-Olefin(e) eingesetzt.

Das erfindungsgemäße Verfahren ist besonders durch den Einsatz von überschüssigem Wasserstoff bezüglich der Gesamtungesättigtheit der einzusetzenden rohen Kohlenwasserstoffströme gekennzeichnet. Die Gesamtungesättigtheit umfaßt den Gehalt an allen olefinischen und acetylenischen Mehrfachbindungen, wobei jede acetylenische Bindung als zwei olefinische Bindungen gerechnet wird. Die Gesamtungesättigtheit kann in bekannter Weise analytisch ermittelt werden und als Mol Gesamtungesättigtheit pro Volumen- oder Gewichtseinheit des Kohlenwasserstoffstroms ausgedrückt werden. Überschüssiger Wasserstoff bedeutet beispielsweise 1,5 bis 20 Mol, bevorzugt 2 bis 8 Mol Wasserstoff pro Mol Gesamtungesättigtheit.

Wasserstoff kann in reiner oder technischer Form eingesetzt werden. Wirtschaftlich vorteilhaft kann ein in petrochemischen Anlagen anfallender, mit Methan und/oder Stickstoff vergesellschafteter Wasserstoff eingesetzt werden. Die $H_2$-Konzentration in einem solchen reinen oder technischen Wasserstoff beträgt 70 bis 100 Vol-% $H_2$, in $H_2$-haltigen Restgasen von petrochemischen Anlagen beträgt sie vielfach 80 bis 90 Vol-% $H_2$.

Ein weiteres besonderes Kennzeichen des erfindungsgemäßen Verfahrens ist der erhöhte Druck, unter dem es durchgeführt wird. Dieser Druck beträgt 12 bis 80 bar, bevorzugt 15 bis 60 bar, besonders bevorzugt 15 bis 35 bar.

Das erfindungsgemäße Verfahren wird zur weitgehenden Hydrierung auch der Monoolefine bei einer tendenziell höheren Temperatur durchgeführt, als sie für die partielle Hydrierung nur der hochungesättigten Verbindungen, etwa nach DE-OS 35 38 564, nötig wäre. Diese Temperatur liegt im Bereich von 60 bis 180°C, bevorzugt 70 bis 140°C, besonders bevorzugt 70 bis 120°C. In ganz besonders bevorzugter Weise wird im Bereich von 80 bis 110°C gearbeitet.

Das erfindungsgemäße Verfahren wird an einem Katalysator oder einem Gemisch von Katalysatoren, der oder das sowohl die Veretherung als auch die Olefinhydrierung bewirkt, durchgeführt. Solche Katalysatoren sind dem Fachmann bekannt. In bevorzugter Weise handelt es sich dabei um heterogene Katalysatoren, von denen das Umsetzungsprodukt leicht abgetrennt werden kann. So kann von einem Katalysator ausgegangen werden, der sowohl saure Gruppen, die die Veretherung bewirken, als auch hydrieraktive Katalysatorbestandteile enthält. Es kann aber auch von einem Katalysatorgemisch ausgegangen werden, dessen einer Bestandteil nur die zur Veretherung nötigen sauren Gruppen trägt, während der andere Bestandteil nur die hydrieraktive Komponente enthält. In jedem Falle ist darauf zu achten, daß die hydrieraktive Komponente oder der Bestandteil eines Katalysatorgemisches, der die hydrieraktive Komponente trägt, nicht in unerwünschter Weise den gebildeten Ether wieder ganz oder teilweise spaltet. Katalysatoren und Gemische von Katalysatoren die hierfür in Frage kommen, sind beispielsweise:

Makroporöse und/oder gelförmige Kationenaustauscher, welche eine hydrieraktive Komponente enthalten und gleichzeitig in der $H^+$-Form vorliegen.

Hydrierkatalysatoren, welche dem Fachmann bekannt sind Pd, Ni, Pt, Rh, Os, Ir etc. auf $Al_2O_3$, $SiO_2$, Spinell, Gemischen daraus oder auf weiteren bekannten Trägern, wenn sie eine genügende Acidität besitzen, um die Veretherungsreaktion zu bewirken.

Auch Gemische aus sauren Ionentauschern mit (gegebenenfalls sauren) Hydrierkatalysatoren sind möglich.

Weiterhin ist es bei einer Fahrweise mit zwei oder mehr Reaktoren möglich, im ersten Reaktor einen Katalysator oder ein Gemisch mehrerer Katalysatoren einzusetzen, der (das) sowohl eine hydrieraktive Komponente hat als auch genügend sauer ist, während im zweiten (in den folgenden) Reaktor(en) Katalysatoren mit lediglich sauren Eigenschaften, beispielsweise Kationenaustauscher in der $H^+$-Form, eingesetzt werden.

In bevorzugter Weise eignen sich für das erfindungsgemäße Verfahren die aus der Veretherung von tert.-Olefinen mit Alkanolen bereits bekannten Kationenaustauscher in $H^+$-Form, wenn sie zusätzlich mit einem oder mehreren Metallen aus der VI. und/oder VII. und/oder VIII. Nebengruppe des Periodensystems der Elemente in elementarer Form beladen sind. Solche Kationenaustauscher können einen makroporösen oder gelförmigen Aufbau haben und sind durch Copolymerisation von Vinylmonomeren und Divinylvernetzern, gegebenenfalls in Gegenwart von Lösungsmitteln, oder durch Kondensation von Phenol und Formaldehyd hergestellt worden. Vinylmonomere sind beispielsweise Styrol oder Acrylsäureester; Divinylvernetzer ist beispielsweise das Divinylbenzol. Der Vernetzungsgrad, gerechnet als Gew.-% an Divinylvernetzer, beträgt 2 bis 65 %, bevorzugt 8 bis 25 %. Saure Gruppen solcher Kationenaustauscher sind beispielsweise Carboxylgruppen, Phosphorsäuregruppen oder Sulfonsäuregruppen. In bevorzugter Weise werden stark saure, Sulfonsäuregruppen enthaltende Kationenaustauscher in der $H^+$-Form eingesetzt. In weiterhin bevorzugter Weise handelt es sich dabei um Styrol-Divinylbenzol-Polymerisate mit solchen Sulfonsäuregruppen, die unter verschiedenen Bezeichnungen handelsüblich sind. Die spezifische Oberfläche solcher Kationenaustauscher für das erfindungsgemäße Verfahren beträgt 5 bis 750 m²/g, bevorzugt 50 bis 250 m²/g, gemessen am trockenen Austauscherharz. Der mittlere Porenradius der Kationenaustauscher beträgt 50 bis 1200 Å, bevorzugt 70 bis 500 Å. Die Kationenaustauscher liegen beispielsweise als Perlpolymerisate mit Korngrößen von 0,1 bis 2 mm oder als Pulverharz in Korngrößen von 10 bis 100 µm vor.

Jedoch können auch abweichende Korngrößen eingesetzt werden. Die Handhabung solcher Kationenaustauscher ist aus der Ionenaustauscher- bzw. Katalysator-Technologie dem Fachmann bekannt.

Als Metalle für die Belegung der Kationenaustauscher seien genannt: Chrom, Molybdän, Wolfram, Mangan, Rhenium, Eisen, Cobalt, Nickel, Ruthenium, Rhodium, Palladium, Osmium, Iridium und Platin. In bevorzugter Weise seien genannt: Palladium, Platin, Rhenium, Molybdän, Nickel, Wolfram und Cobalt; in besonders bevorzugter Weise seien genannt: Palladium, Platin und Nickel. Diese Metalle können einzeln oder als Gemisch mehrerer von ihnen auf den Kationenaustauscher gebracht werden. In bevorzugter Weise wird nur eines der genannten Metalle eingesetzt.

Die Belegung der Kationenaustauscher mit einem oder mehreren der genannten Metalle kann beispielsweise so erfolgen, daß ein nicht-komplexes, kationisches Salz dieser Metalle mit dem Kationenaustauscher in der $H^+$-Form in an sich bekannter Weise zusammengebracht wird. Hierbei zieht das Metallkation in ionenaustauschenderweise auf den Kationenaustauscher auf. Die hierbei freiwerdende Säure wird gegebenenfalls durch geeignete, alkalisch reagierende Verbindungen, beispielsweise Alkalihydroxidlösung neutralisiert. Die Menge des aufzubringenden Metallsalzes wird berechnet oder durch einfache Vorversuche bestimmt, so daß die gewünschte Menge an Metallen auf den Kationenaustauscher aufgebracht wird. Diese Menge beträgt 0,001 bis 10 g, bevorzugt 0,2 bis 3 g, bezogen auf 1 l trockenen Kationenaustauscher.

Der metalldotierte Kationenaustauscher wird neutral gewaschen, getrocknet, beispielsweise bei 80 bis 100°C unter Vakuum, und anschließend zur Überführung der aufgebrachten Metalle in den elementaren Zustand mit Wasserstoff behandelt, beispielsweise bei 2 bis 50 bar, vorzugsweise bei 10 bis 30 bar $H_2$-Druck und einer Temperatur von 50 bis 150°C, vorzugsweise 70 bis 120°C. Grundsätzlich können auch andere Reduktionsmittel, wie Hydrazin, Formaldehyd oder Ameisensäure, benutzt werden.

Ein solcher metallbeladener Kationenaustauscher in der $H^+$-Form enthält gleichzeitig die sauren Gruppen zur Veretherung und die hydrieraktive Komponente. Diese bevorzugt eingesetzten metallbeladenen Kationenaustauscher in der $H^+$-Form ergeben eine höhere Ausbeute an Alkyl-tert.-alkyl-ethern als dem Gehalt an tert.-Olefinen im umzusetzenden rohen Kohlenwasserstoffstrom entspricht. Dies ist nur dadurch erklärlich, daß verzweigte, jedoch nicht tertiäre Olefine einer Isomerisierung unterliegen, die die Doppelbindung an die Verzweigungsstelle verschiebt, so daß ein tert.-Olefin entsteht, das nunmehr ebenfalls in die Veretherungsreaktion eintreten kann. Diese überraschende Verfahrensvariante unter Benutzung eines metallbeladenen Kationenaustauschers in der $H^+$-Form zeigt, daß bei der weitgehenden Hydrierung auch der Monoolefine (neben der Hydrierung der hochungesättigten Verbindungen) nicht nur die tert.-Olefine vor der Mithydrierung verschont bleiben, sondern daß zusätzlich verzweigte Olefine, die zu tert.-Olefinen isomerisiert werden können ebenfalls vor der Hydrierung verschont bleiben und damit eine Isomerisierung und anschließende Veretherung in der beschriebenen Weise eingehen können.

Der beschriebene metalldotierte Kationenaustauscher in der $H^+$-Form kann auch im Gemisch mit einem nicht mit Metallen beladenen Kationenaustauscher in der $H^+$-Form abgemischt und als solches Gemisch erfindungsgemäß eingesetzt werden. Die Zumischung eines nicht mit Metallen beladenen Kationenaustauschers in der $H^+$-Form kann in einem weiten Bereich von 1 bis 90 Vol.-%, bezogen auf das Gemisch aus dem beladenen und dem nicht-beladenen Kationenaustauscher, durchgeführt werden. Eine solche Abmischung dient vor allem der Kostensenkung, denn der mit Metallen beladene Kationenaustauscher ist beträchtlich teurer als der nicht-beladene. Es ist daher zur Erzielung dieses Zweckes sinnvoll, beispielsweise 40 bis 90 Vol.-%, bevorzugt 55 bis 75 Vol.-%, bezogen auf das Gesamtgemisch, an dem nicht mit Metallen beladenen Kationenaustauscher in der $H^+$-Form einzusetzen.

Der Katalysator oder das Katalysatorgemisch wird einer Belastung von a = 0,1 bis 100, bevorzugt 0,3 bis 20, besonders bevorzugt 0,5 bis 5 unterworfen. Das Zeichen "a" bedeutet hierbei Kilogramm rohes, tertiäre Olefine, Diolefine, Monoolefine und Paraffine enthaltendes Kohlenwasserstoffgemisch pro Liter Katalysatorvolumen pro Stunde.

Im erfindungsgemäßen Verfahren liegen der rohe Kohlenwasserstoffstrom und das Alkanol in der flüssigen Phase vor. Der Katalysator oder das Katalysatorgemisch werden im Festbett, beispielsweise in einem Schachtreaktor oder in einem Röhrenreaktor angeordnet. Die flüssigen Einsatzstoffe können hierbei das Katalysatorbett von unten nach oben oder umgekehrt anströmen. Der Wasserstoff wird den flüssigen Einsatzstoffen im Gleichstrom oder im Gegenstrom zugeführt. Eine Führung sowohl der flüssigen Einsatzstoffe als auch des Wasserstoffs im Gleichstrom ist hierbei bevorzugt.

Die Hydrierung ist eine exotherme Reaktion, was im erfindungsgemäßen Verfahren, bei dem nicht nur eine weitgehende Hydrierung auch aller Monoolefine angestrebt wird, sondern diese weitgehende Hydrierung der Monoolefine auch bei einem tendenziell höheren Temperaturniveau, verglichen mit der Hydrierung nur der hochungesättigten Verbindungen, durchgeführt werden muß, zur genauen Kontrolle der Temperaturführung zwingt. Hierzu kann es günstig sein und stellt auch eine bevorzugte Variante dar, das erfindungsgemäße Verfahren in mehreren hintereinanderliegenden, bevorzugt in zwei Reaktoren durchzuführen. Zusätzlich kann ein Teil des vom ersten Reaktor abströmenden Reaktionsgemisches abgenommen und zum Reaktoreingang dieses ersten Reaktors zurückgeführt werden, während der nicht abgenommene Teil des Reaktionsgemisches in einen zweiten oder in weitere Reaktoren geleitet wird, Der zum Reaktoreingang zurückgeführte abgenommene Teil des Reaktionsgemisches kann auf dem Rückführungswege durch einen Kühler geführt werden, um Reaktionswärme aus dem System zu nehmen.

Eine weitere vorteilhafte Variante der Reaktionsführung besteht darin, den Wasserstoff an verschiedenen Stellen des Reaktors oder in verschiedene Reaktoren eines aus mehreren Reaktoren bestehenden Reaktorsystems einzuspeisen. Weiterhin kann es vorteilhaft sein, insbesondere mit einer Aufteilung der Wasserstoffeinspeisung, Katalysatorgemische, falls solche benutzt werden, in verschiedener Zusammensetzung in einzelne Abschnitte eines Reaktors oder in verschiedene Reaktoren, falls in mehreren Reaktoren gearbeitet wird, in unterschiedlicher Zusammensetzung bezüglich der hydrieraktiven Komponente und der sauren Komponente zu verwenden. So kann es beispielsweise bei Verwendung eines Gemisches aus mit Metallen beladenem Kationenaustauscher und nicht mit Metallen beladenem Kationenaustauscher günstig sein, bei mehreren hintereinandergeschalteten Reaktoren im ersten zunächst einen geringeren Anteil des mit Metallen beladenen Kationenaustauschers im Gemisch einzusetzen als im zweiten oder in den folgenden Reaktoren.

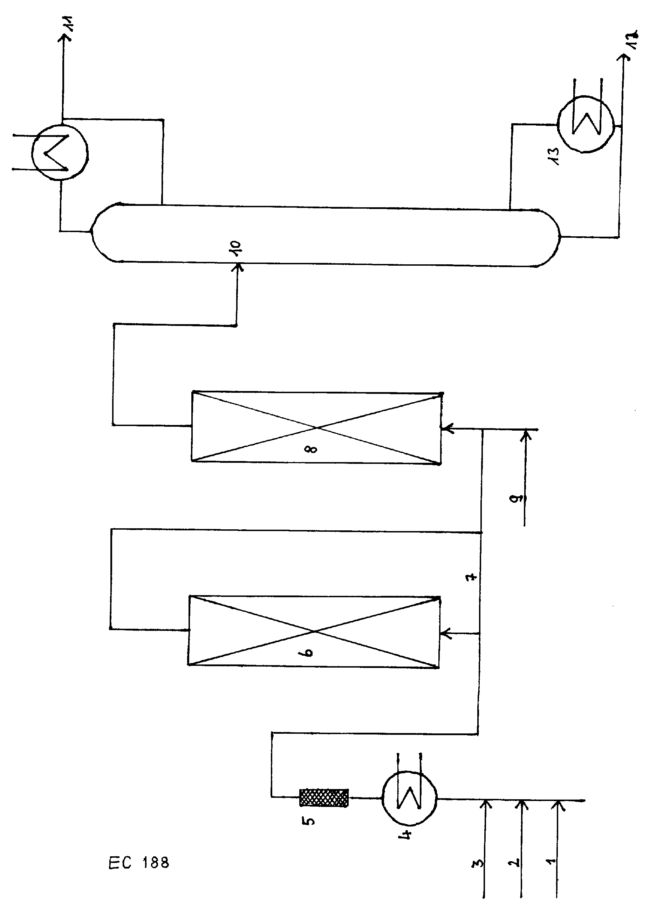

Das erfindungsgemäße Verfahren sei anhand der beigefügten Fig. 1 beispielhaft wie folgt erläutert: Das rohe Kohlenwasserstoffgemisch (1), der (die) Alkanol(e) (2) und Wasserstoff (3) werden als Einsatzströme nach Durchlaufen eines Vorwärmers (4) und eines Mischers (5) dem Reaktor (6), der zumindest teilweise mit dem mit Metallen beladenen Kationenaustauscher in der $H^+$-Form beschickt ist, zugeführt und dort umgesetzt. Nach Verlassen des Reaktors (6) kann ein Teil des Reaktionsproduktes zur Wärmeabgabe recyclisiert werden (7). Nach Durchlaufen eines zweiten Reaktors (8), in welchem gegebenenfalls weiterer Wasserstoff (9) zugegeben werden kann, gelangt das Reaktionsprodukt in die Stabilisierungskolonne (10). In (10) wird über Kopf Restgas, vorwiegend überschüssiger Wasserstoff und gegebenenfalls vorhandenes Methan oder Stickstoff, als Strom (11) abgezogen. Die beiden Reaktoren (6) und (8) können baulich auch zusammengefaßt werden und als ein Reaktorsystem betrieben werden. (10) wird durch einen Umlauferhitzer (13) beheizt. Ein Teil des umlaufenden Sumpfstromes wird als gumfreier, olefinarmer, dampfdruckerniedrigter, farbloser, Alkyl-tert.-alkyl-ether enthaltender Kraftstoff oder Kraftstoffkomponente (12) abgezogen. Für einen solchen Kraftstoff bzw. eine solche Kraftstoffkomponente hat sich der Sprachgebrauch "Green Gasoline" gebildet.

Die erfindungsgemäß herstellbaren Kraftstoffe und Kraftstoffkomponenten zeichnen sich durch eine restliche Bromzahl als Ausdruck der restlichen Ungesättigtheit von 5 bis 40, bevorzugt 10 bis 30 g $Br_2$/100 g aus, während die eingesetzten rohen Kohlenwasserstoffströme Bromzahlen von etwa 100 und darüber haben. Der höhere Anteil an Paraffinen, verglichen mit dem rohen Kohlenwasserstoffstrom als Einsatzmaterial zeigt sich in einer Verminderung des Dampfdruckes. Der Umfang der Oktanzahlerhöhung ist selbstverständlich in einer dem Fachmann geläufigen Weise von der Menge der veretherbaren tert.-Olefine im rohen Kohlenwasserstoffstrom abhängig und ferner vom Umfang der tatsächlich durchgeführten Veretherung, der u.a. von der molaren Menge an Alkanol abhängt. Das Ausmaß der Verringerung der Ungesättigtheit (gekennzeichnet durch die Abnahme der Bromzahl) ist abhängig von der Einstellung der Temperatur, der Wasserstoffmenge und des Druckes und ist dem Fachmann bekannt.

Die Ausbeutesteigerung an Alkyl-tert.-alkyl-ether durch Isomerisierung verzweigter, jedoch nicht tertiärer Olefine und die Verringerung der Sensitivity sind bereits oben erwähnt worden. Bei einem Gehalt an veretherbaren Olefinen im rohen Ausgangskohlenwasserstoff von 10 bis 30 Gew.-% ist ein Gehalt an Alkyl-tert.-alkyl-ether von etwa 12 bis 42 Gew.-% im erfindungsgemäßen Umsetzungsgemisch zu erwarten. Die erwähnte Isomerisierung der nicht tertiären, verzweigten Olefine erfolgt unter den angegebenen Bedingungen des erfindungsgemäßen Verfahrens in der Weise, daß die Etherausbeute je nach Konzentration an solchen Verbindungen im rohen Kohlenwasserstoffgemisch um 1 bis 10 Prozentpunkte angehoben werden kann. Die Sensitivity sinkt von etwa 14 bis 16 auf 10 bis 12, und die Farbzahl nach APHA ist stets unter 8, vielfach unter 5 und kann Werte unter 4 erreichen.

Weiterhin enthalten die erfindungsgemäß hergestellten Kraftstoffe und Kraftstoffkomponenten nur wenig Gum-Bildner, so daß die sichere Einhaltung und sogar Unterschreitung der in DIN 51 607 und in DIN 51 600 bzw. in der ASTM-Norm für Automotive Gasoline festgelegten Höchstmenge für Gum (gemessen als Abdampfrückstand) von 5 mg/100 ml gewährleistet ist. Infolge des jederzeit vorhandenen Überschusses an Wasserstoff bei der erfindungsgemäßen Herstellung von Kraftstoffen und Kraftstoffkomponenten und der damit verhinderten Gum-Bildung wird nicht nur die Standzeit des Katalysators, insbesondere die Standzeit des mit Metallen beladenen Kationenaustauschers in der $H^+$-Form, wesentlich verlängert, sondern durch die damit gleichzeitig verhinderte Belegung der aktiven Zentren des Katalysators wird ein gleichbleibend hoher Umsatz über eine lange Zeit erreicht. So wird die Standzeit eines mit Metallen beladenen Kationenaustauschers in der $H^+$-Form von etwa 8 Monaten ohne Mitverwendung von Wasserstoff auf bisher erreichte mindestens 2 Jahre verlängert, wobei dieser Katalysator immer noch im Einsatz ist.

Die erfindungsgemäß erhaltenen Kraftstoffe und Kraftstoffkomponenten mit einem Anteil an Alkyl-tert.-alkyl-ether können, falls dies gewünscht wird, in einer dem Fachmann bekannten Weise destillativ aufgetrennt werden, beispielsweise um die hierbei erhältlichen Teile der Kraftstoffe und Kraftstoffkomponenten separat und gezielt anderen Kraftstoffen oder Kraftstoffkomponenten oder auch anderen Verwendungen zuführen zu können. Bei einer destillativen Aufarbeitung können beispielsweise folgende Anteile der erfindungsgemäß hergestellten Umsetzungsprodukte gewonnen werden (a) das Restgas und die Kohlenwasserstoffe als Kopfstrom; (b) einen hauptsächlich den Alkyl-tert.-alkyl-ether (beispielsweise TAME aus einem $C_5$-Schnitt) enthaltenden Strom als Seitenstrom und (c) einen Gum-freien Sumpfstrom, der höhere Ether und andere Hochsieder enthält.

### Beispiel 1

Herstellung eines erfindungsgemäß einzusetzenden Katalysatorgemisches aus Kationenaustauschern

Einem handelsüblichen Styrol-Divinyl-Copolymer mit Sulfonsäuregruppen (Lewatit SPC 118® der Bayer AG) wurde in der wasserfeuchten $H^+$-Form soviel Palladiumacetat angeboten, daß 1 g Pd pro Liter trockenes Harz nach Reduktion mit $H_2$ auf dem Kationenaustauscher vorhanden waren. Die bei der Behandlung mit Palladiumacetat freigesetzte Säure wurde mit 1 gew.-%iger NaOH neutralisiert. Der neutral gewaschene Kationenaustauscher wurde 24 Stunden bei 100°C in Wasserstrahlpumpen Vakuum getrocknet. Das auf dem Kationenaustauscher befindliche Palladium wurde bei 90 bis 100°C und 20 bis 25 bar $H_2$-Druck innerhalb von 48 Stunden zum Metall reduziert. Der so hergestellte mit Pd beladene Kationenaustauscher wurde im Gemisch mit nicht mit Metall beladenem Kationenauslauscher (ebenfalls Lewatit SPC 118®) im Volumenverhältnis 1:1 als Katalysatorsystem eingesetzt.

### Beispiel 2

Das nach Beispiel 1 erhaltene Katalysatorgemisch wurde in einer temperierbaren, aus 2 Doppelmantelreaktoren bestehenden Durchlauflaborapparatur in nachfolgender Weise eingesetzt: 200 ml Pd-haltiger stark saurer Kationenaustauscher wurde in den ersten Durchlaufreaktor mit 25 ml lichter Weite und

7

Temperaturmeßstellen im Abstand von 100 mm eingefüllt; in gleicher Weise wurde der dimensionsgleiche nachgeschaltete 2. Reaktor mit nicht dotiertem saurem Kationenaustauscher beschickt. Als rohes Kohlenwasserstoffgemisch wurde der $C_5$-Strom eines Steam-Crackers (sogenannter Aromatenvorlauf) mit einem Gehalt von 11,6 Gew.-% an 2-Methylbuten-2, 2,6 Gew.-% an 2-Methylbuten-1 (beides tertiäre Olefine) und 1,1 Gew.-% an 3-Methylbuten-1 (nicht tertiäres Olefin) eingesetzt. Das Einsatzprodukt enthielt neben $C_5$-Kohlenwasserstoffen zusätzlich einen Anteil von etwa 21 Gew.-% $C_6$-Kohlenwasserstoffen. Die Bromzahl dieses Einsatzproduktes lag bei 105, die MOZ bei 77, die ROZ bei 91. Der Siedebereich lag bei 35 bis 65°C; der Dampfdruck bei 38°C (Reid Vapour Pressure = RVP) lag bei 0,97 bar. Der Diolefingehalt betrug 0,38 %. Die Belastung des Katalysators wurde zu a = 1 und der Druck auf 22 bar eingestellt. Die Temperatur des ersten Reaktors wurde bei 90 bis 95°C, die des zweiten Reaktors bei 65°C gehalten. Das Kohlenwasserstoffgemisch wurde mit der stöchiometrischen Menge an Methanol, bezogen auf veretherbare Olefine, in einer Mischkammer vor dem Reaktor gemischt und vorgewärmt. Die zugegebene Menge an Wasserstoff wurde so eingestellt, daß eine Abgasentstehung von 160 l/h pro Liter Katalysator eingehalten wurde.

Als Reaktionsprodukt wurde eine farblose, praktisch diolefinfreie Ether enthaltende Kraftstoffkomponente erhalten: Farbzahl nach APHA <5; Diene <0,01 Gew.-%; 14,0 Gew.-% TAME und 1,5 Gew.-% höhere tert. Ether. 0,5 Gew.-% des 3-Methylbuten-1 wurde durch Isomerisierung umgewandelt und verethert. Der olefinische Anteil wurde um mehr als zwei Drittel abgesenkt, wodurch sich eine $Br_2$-Zahl von 34 ergab. Die MOZ wurde auf 81,4, die ROZ auf 92,4 gesteigert, woraus sich eine Sensitivity von 11 ergab. Der Dampfdruck betrug 0,84 bar (RVP); der Abdampfrückstand betrug 1 mg/100 ml.

### Beispiel 3

Der Versuch wurde wie im Beispiel 2 durchgeführt, das Reaktionsprodukt wurde jedoch schon nach dem 1. Veretherungsreaktor abgezogen, welcher ausschließlich mit dem Pd-dotierten Kationenaustauscher beschickt war; die Belastung wurde zu a = 1 eingestellt. Es wurde ein dem Beispiel 2 vergleichbares Reaktionsprodukt erhalten.

### Beispiel 4

Der Versuch wurde wie im Beispiel 2 durchgeführt, jedoch erfolgte die Aufteilung des Katalysatorsystems in der Art, daß jeweils die Hälfte der beiden Reaktoren im unteren Teil mit Pd-dotiertem Kationenaustauscher und die andere Hälfte mit nicht dotiertem Ionenaustauscher beschickt wurde. Zur günstigeren Verteilung der Reaktionswärme wurde der Wasserstoff im Verhältnis 1:1 den beiden Reaktoren zugeführt. Auch bei dieser Fahrweise wurde ein dem Beispiel 2 entsprechendes Reaktionsprodukt erhalten.

### Beispiel 5 (zum Vergleich)

Der Versuch wurde wie im Beispiel 2 durchgeführt, jedoch ohne Zugabe von $H_2$. Das 13,4 Gew.-% TAME enthaltende Reaktionsprodukt war gelb gefärbt, besaß eine Farbzahl nach APHA von ca. 2500 und einen Abdampfrückstand von 20 mg/100 ml. Die Oktanzahlwerte lagen unter denen aus Beispiel 2. Dampfdruck und Olefingehalt ($Br_2$-Zahl = 80) lagen über den Werten aus Beispiel 2.

### Beispiel 6 (zum Vergleich)

Der Versuch wurde wie im Beispiel 2 durchgeführt, jedoch wurde die eingesetzte Wasserstoffmenge auf 20 l $H_2$ pro Liter Katalysator eingestellt, die Reaktortemperaturen betrugen 75°C im 1. und 70°C im 2. Reaktor. Der Druck wurde auf 15 bar eingestellt. Als Reaktionsprodukt wurde eine farblose Kraftstoffkomponente mit 14,0 Gew.-% TAME erhalten. Die Farbzahl nach APHA war <5, der Abdampfrückstand betrug 1 mg/100 ml. Die Bromzahl lag jedoch bei etwa 80; der Dampfdruck lag 0,9 (RVP); der MOZ-Wert lag bei 79,5, der ROZ-Wert lag bei 92,8, woraus sich eine Sensitivity von 13,3 ergab.

### Patentansprüche

1. Verfahren zur Herstellung von hochoktanigen, olefinarmen Kraftstoffen und Kraftstoffkomponenten mit einem Gehalt an Alkyl-tert.-alkyl-ethern, dadurch gekennzeichnet, daß man einen rohen Kohlenwasserstoffstrom, der $C_5$-$C_8$-tert.-Olefine, bevorzugt $C_5$-$C_7$-tert.-Olefine, neben gesättigten und olefinisch, diolefinisch und acetylenisch ungesättigten Kohlenwasserstoffen im gleichen Siedebereich enthalt, gleichzeitig mit einem oder mehreren $C_1$-$C_4$-Alkanolen, bevorzugt $C_1$-$C_2$-Alkanolen, besonders bevorzugt Methanol, in einer Menge von 0,5 bis 5 Mol, bevorzugt 0,8 bis 2,5 Mol, besonders bevorzugt 1 bis 2 Mol, pro Mol des oder der tert.-Olefine und mit 1,5 bis 20 Mol pro Mol der Gesamtungesättigtheit an Wasserstoff unter einem Druck von 12 bis 80 bar, bevorzugt 15 bis 60 bar, besonders bevorzugt 15 bis 35 bar, und bei einer Temperatur von 60 bis 180°C, bevorzugt 70 bis 140°C, besonders bevorzugt 70 bis 120°C, an einem an sich bekannten Katalysator oder einem Gemisch an sich bekannter Katalysatoren, der oder das sowohl die Veretherung als auch die Olefinhydrierung bewirkt, umsetzt.

2. Verfahren nach Anspruch 1, dadurch gekennzeichnet, daß als Katalysator ein gelförmiger oder makroporöser Kationenaustauscher in der $H^+$Form eingesetzt wird, der 0,001 bis 10 g eines oder mehrerer Metalle der VI. und/oder VII. und/oder VIII. Nebengruppe des Periodensystems der Elemente in elementarer Form pro Liter trockenen Kationenaustauschers enthält und einen Vernetzungsgrad von 2 bis 65 %, bevorzugt 8 bis 25 % sowie eine spezifische Oberflache von 5 bis 750 $m^2$/g trockenes Austauscherharz aufweist.

3. Verfahren nach Anspruch 2, dadurch gekennzeichnet, daß der Kationenaustauscher eines oder mehrerer Metalle aus der Gruppe Palladium, Platin, Rhenium, Molybdän, Nickel, Wolfram oder Kobalt, bevorzugt aus der Gruppe Palladium, Platin und Nickel, enthalt.

4. Verfahren nach Anspruch 1, dadurch gekennzeichnet, daß Wasserstoff in einer Menge von 2 bis 8 Mol pro Mol Gesamtungesattigtheit eingesetzt wird.

5. Verfahren nach Anspruch 2, dadurch gekennzeichnet, daß der mit Metallen beladene Kationenaustauscher in der $H^+$-Form im Gemisch mit einem nicht mit Metallen beladenen Kationenaustauscher in der $H^+$-Form eingesetzt wird.

6. Verfahren nach Anspruch 1, dadurch gekennzeichnet, daß in mehreren hintereinanderliegenden, bevorzugt in 2 Reaktoren gearbeitet wird.

## Claims

1. Process for producing high-octane, low-olefin motor fuels and motor fuel components having a content of alkyl tert.-alkyl ethers, characterized in that the crude hydrocarbon stream, which contains tertiary $C_5$-$C_8$-olefins, preferably tertiary $C_5$-$C_7$-olefins, in addition to saturated and olefinically, diolefinically and acetylenically unsaturated hydrocarbons in the same boiling range, is reacted simultaneously with one or more $C_1$-$C_4$-alkanols, preferably $C_1$-$C_2$-alkanols and particularly preferably methanol, in a quantity from 0.5 to 5 mol, preferably 0.8 to 2.5 mol and particularly preferably 1 to 2 mol, per mole of the tertiary olefin(s), and with 1.5 to 20 mol per mole of the total unsaturation of hydrogen under a pressure from 12 to 80 bar, preferably 15 to 60 bar and particularly preferably 15 to 35 bar, and at a temperature from 60 to 180°C, preferably 70 to 140°C and particularly preferably 70 to 120°C, on a catalyst known per se or on a mixture of catalysts known per se, which effect(s) both the etherification and the olefin hydrogenation.

2. Process according to Claim 1, characterized in that the catalyst used is a gel-type or macroporous cation exchanger in the $H^+$ form, which contains 0.001 to 10 g of one or more metals of subgroups VI and/or VII and/or VIII of the periodic table of the elements in the elemental form per litre of dry cation exchanger and has a degree of crosslinking from 2 to 65%, preferably 8 to 25%, and a specific surface area from 5 to 750 $m^2$/g of dry exchanger resin.

3. Process according to Claim 2, characterized in that the cation exchanger contains one or more metals from the group comprising palladium, platinum, rhenium, molybdenum, nickel, tungsten or cobalt, preferably from the group comprising palladium, platinum and nickel.

4. Process according to Claim 1, characterized in that hydrogen is employed in a quantity of from 2 to 8 mol per mole of total unsaturation.

5. Process according to Claim 2, characterized in that the cation exchanger in the $H^+$ form, loaded with metals, is employed as a mixture with a cation exchanger in the $H^+$ form, not loaded with metals.

6. Process according to Claim 1, characterized in that it is operated in a plurality of series-arranged reactors, preferably in 2 reactors.

## Revendications

1. Procédé de fabrication de carburants et composants de carburants à indice d'octane élevé, comportant peu d'oléfines, avec une teneur en éthers d'alkyle et alkyle tertiaire, caractérisé en ce que l'on fait réagir un courant brut d'hydrocarbures qui contient des oléfines tertiaires en $C_5$-$C_8$, de préférence des oléfines tertiaires en $C_5$-$C_7$, en plus des hydrocarbures saturés et insaturés oléfiniques, dioléfiniques et acétyléniques du même domaine d'ébullition, en même temps qu'un ou plusieurs alcanols en $C_1$-$C_4$, de préférence des alcanols en $C_1$-$C_2$, en particulier du méthanol à raison de 0,5 à 5 moles, de préférence 0,8 à 2,5 moles, en particulier de 1 à 2 moles, par mole de la ou des oléfines tertiaires et avec 1,5 à 20 moles d'hydrogène par mole de l'insaturation totale, sous une pression de 12 à 80 bar, de préférence de 15 à 60 bar, en particulier de 15 à 35 bar et à une température de 60 à 180°C, de préférence de 70 à 140°C, en particulier de 70 à 120°C, sur un catalyseur connu en soi ou un mélange de catalyseurs connus en soi produisant tant l'éthérification que l'hydrogénation des oléfines.

2. Procédé selon la revendication 1, caractérisé en ce que l'on utilise comme catalyseur un échangeur de cations sous la forme $H^+$, en forme de gel ou macroporeux, qui contient 0,001 à 10 g d'un métal ou de plusieurs métaux, sous forme élémentaire, des groupes secondaires VI et /ou VII et/ou VIII du système périodique de classification des éléments par litre d'échangeur de cations sec et présente un degré de réticulation de 2 à 65%, de préférence de 8 à 25%, ainsi qu'une surface spécifique de 5 à 750 m²/g de résine échangeuse sèche.

3. Procédé selon la revendication 2, caractérisé en ce que l'échangeur de cations contient un ou plusieurs métaux du groupe comportant le palladium, le platine, le rhénium, le molybdène, le nickel, le tungstène ou le cobalt, de préférence du groupe comportant le palladium, le platine et le nickel.

4. Procédé selon la revendication 1, caractérisé en ce que l'hydrogène est utilisé en quantités de 2 à 8 moles par mole d'insaturation totale.

5. Procédé selon la revendication 2, caractérisé en ce que l'échangeur de cations sous la forme $H^+$, dopé avec des métaux, est utilisé en mélange avec un échangeur de cations sous la forme $H^+$ non dopé avec des métaux.

6. Procédé selon la revendication 1, caractérisé en ce que l'on travaille dans plusieurs réacteurs, de préférence dans deux réacteurs en cascade.

EC 188